(19) Europäisches Patentamt / European Patent Office / Office européen des brevets

(11) **EP 1 875 941 A1**

(12) **EUROPEAN PATENT APPLICATION**

(43) Date of publication:
**09.01.2008 Bulletin 2008/02**

(51) Int Cl.:
*A61M 25/09* (2006.01)

(21) Application number: **07251897.0**

(22) Date of filing: **08.05.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **03.07.2006 JP 2006183925**

(71) Applicant: **ASAHI INTECC CO., LTD.**
**Nagoya-shi,**
**Aichi-ken (JP)**

(72) Inventor: **Kato, Tomihisa**
**Nagoya-shi**
**Aichi-ken (JP)**

(74) Representative: **Senior, Alan Murray**
**J.A. KEMP & CO.**
**14 South Square**
**Gray's Inn**
**London WC1R 5JJ (GB)**

(54) **A medical guide wire, an assembly body of the same medical guide wire and microcatheter, an assembly body of the same medical guide wire, a balloon catheter and a guiding catheter**

(57) A medical guide wire (1) has a helical spring body (3), a distal end portion of which has a radiopaque helical portion (31), and an elongate core (2) placed within the helical spring body (3). The elongate core (2) has a distal end portion (21) diametrically thinned and having a proximal end portion (22) diametrically thickened. A securement portion (10) is provided in which the distal end of the helical spring body (3) and the distal end of the elongate core (2) are air-tightly bonded. A floatage chamber (5) is formed within the radiopaque helical portion (31). A synthetic resin layer (4) coated on an outer surface of the helical spring body (3) forms a cylindrical film (43) at a clearance (C) between neighboring coil lines (W) of the helical spring body (3) in a condition that an outer diameter of the cylindrical film (43) is smaller than that of the helical spring body (3) when expanding the helical spring body (3) in the moistened condition, thus enabling an operator to a deep insertion against a blood vessel due to a buoyant property and an increased pressure resistance caused by blood streams.

Fig. 1

EP 1 875 941 A1

**Description**

The invention relates to a medical guide wire which is improved to enable an operator to a deep insertion into a tortuous blood vessel especially by making a best use of blood streams.

Upon therapeutically diagnosing human organs in particular such as a blood vessel, digestive tract, urethral canal or the like, it is necessary to insert a guide wire to a destination before inserting a catheter into the blood vessel. In order to smoothly insert the guide wire into a sinuously curved blood vessel (areas difficult to reach). There have been various techniques thus far introduced.

By way of illustration, Japanese Laid-open Patent Application No. 2000-135289 discloses a guide wire which has a radiopaque helical metal coil fixed to a distal end portion of an elongate core. A synthetic resin tube is provided to surround an outer surface of the helical metal coil and cover the outer surface of the helical metal coil when swelled. This enables the operator to achieve a good lubricity due to a smoothness of the synthetic resin tube, while at the same time, reducing the thrombi deposited on the synthetic resin tube with a good pushability insured by a thinned distal end of the elongate core when inserting it into the blood vessel.

Japanese Laid-open Patent Application No. 4-9162 discloses a guide wire having a flexible property at a distal end portion and a rigid property at a main portion. A highly radiopaque metal (highly sensitive as a contrast medium for X-rays photography) is inserted into the distal end portion. A synthetic resin covers the guide wire, and exhibits the lubricity when moistened, so as to enable the operator to a good steerability (push and pull) because of the high lubricity.

Japanese Utility Model Registration No. 2588582 discloses a guide wire in which a radiopaque coil is fixed to a distal end portion of an elongate core. A hydorophilic layer and a hydrophobic layer are coated on the guide wire so as to achieve a good maneuverability because of a decreased friction at a proximal end section.

With the above prior arts in mind, there are no appreciable idea to enable the operator to a good steerability by making not only a good use of buoyance in the blood or blood streams but also utilizing a pressure resistance and difference of the specific gravity between the double-layered structure in the blood streams.

Therefore, it is an object of the invention to overcome the above drawbacks, and provide a medical guide wire which is capable to improve a steerability by forming a floatage chamber inside a distal end portion of a helical spring portion which is liable to sag under the influence of gravity.

It is another object of the invention to provide a medical guide wire which is capable to maintain an air-tightness when a helical spring portion is expanded to enlarge a clearance between coil lines of the helical spring portion, and strengthening a forward propelling force by utilizing a pressure resistance and difference of the specific gravity between a hydrophobic layer and a hydrophilic layer in the blood streams.

SUMMARY OF THE INVENTION

According to the invention, there is provided a medical guide wire in which a helical spring body is provided, a distal end portion of which has a radiopaque helical portion made by a radiopaque material, and a proximal end of which has a radiotransparent helical portion made by a radiotransparent material. An elongate core is placed within the helical spring body, and having a distal end portion diametrically thinned and having a proximal end portion diametrically thickened. A distal end of the helical spring body and a distal end of the elongate core are air-tightly bonded. A synthetic resin layer is air-tightly coated on an outer surface of the helical spring body in a circumferential direction. A hermetical wall is provided at a proximal end of the radiopaque helical portion so as to air-tightly bond the helical spring body and the elongate core together. A floatage chamber is provided within the radiopaque helical portion by the hermetical wall, the synthetic resin layer and a securement portion in which the distal end of the helical spring body and the distal end of the elongate core are air-tightly bonded. The synthetic resin layer is lubricous in moistened condition more than in desiccated condition in which the synthetic resin layer forms a cylindrical film at a clearance between neighboring coil lines of the helical spring body in a condition that an outer diameter of the cylindrical film is smaller than that of the helical spring body when expanding the helical spring body in the moistened condition, so that the clearance between the neighboring coil lines of the helical spring body is 50-100 % of an outer diameter of the coil lines of the helical spring body. The cylindrical film resides between an inner surface of the helical spring body and a central diameter portion of the helical spring body, so that an outer surface of the helical spring body undulates to form a concave-convex portion with the neighboring coil lines running as a convex portion and the cylindrical film deformed as a concave portion, so as to strengthen a forward propelling force due to a pressure resistance caused by blood streams colliding against the convex portion together with spiral currents caused by a coil-winding configuration of the helical spring body portion when inserting the helical spring body into a blood vessel.

With the floatage chamber selectively provided within the distal end portion of the radiopaque helical portion of the helical spring body, the floatage chamber is subjected to a buoyance due to the blood or blood streams so as to prevent the distal end portion of the helical spring body from sagging under the influence of gravity.

This enables the operator to maintain a stable posture of the distal end portion of the medical guide wire in the blood vessel, so as to enhance the steerability of the medical guide wire.

With the cylindrical film provided by expanding the synthetic resin layer in the moistened condition, it is possible to maintain an air-tightness within the helical spring body without escaping the gaseous component from the helical spring body when the radiopaque helical portion is manipulatively bent to broaden the clearance between the coil lines of the helical spring portion.

With the clearance between the neighboring coil lines of the helical spring body as 50-100 % of the outer diameter of the coil lines of the helical spring body, it is possible to maintain the air-tightness within the helical spring body so as to secure the elastical restitution force due to the floatage chamber when the helical spring body inverts the distal end portion in the blood vessel by way of example. This is because the cylindrical film fills the clearance between the neighboring coil lines of the helical spring body.

At the time of inserting the medical guide wire into the blood vessel, the helical spring body is subjected to the pressure resistance and the spiral currents (forward propelling force) due to the convex-concave portion, thus enabling the operator to the deep insertion into the blood vessel.

According to other aspect of the invention, an elastical restitution force is utilised which develops by a pneumatic pressure increased within the floatage chamber upon bending the radiopaque helical portion into a bent position, and the pneumatic pressure decreased to an original quantity level within the floatage chamber upon releasing the radiopaque helical portion from the bent position.

With the floatage chamber selectively provided within the radiopaque helical portion which is readily subjected to plastic deformations, it is possible to significantly reduce the sagging amount and the plastic deformation of the radiopaque helical portion by utilizing the elastical restitution force of the floatage chamber. This enables the operator to stably maintain an initial good configuration of the distal end portion of the medical guide wire.

According to other aspect of the invention, the synthetic resin layer is made from a mixture of a hydrophilic polymer and a hydrophobic polymer. The synthetic resin layer has a specific gravity decreasing from an inner side to an outer side of the synthetic resin layer when it is moistened.

Except for the mixture in which the hydrophilic polymer and the hydrophobic polymer are simply mixed, the hydrophilic polymer may be added to the mixture of the hydrophobic polymer and an adhesive polymer so as to form another mixture. Otherwise, the hydrophilic polymer may be mixed with the hydrophobic polymer, to which a plasticizer is added so as to form still another mixture. Alternatively, the hydrophilic polymer may be mixed with the hydrophobic polymer, to which the mixture of the adhesive polymer and the plasticizer is added so as to form yet another mixture.

With the weight ratio of the hydrophilic polymer successively increasing from the inner side to the outer side in which the synthetic resin layer progressively increases its volume, the outer side portion of the hydrophilic polymer occupies a larger portion of the synthetic resin layer, thus making it possible to produce a lightweight guide wire with a high buoyant property insured because the outer side portion of the hydrophilic polymer has a smaller specific gravity.

According to other aspect of the invention, the synthetic resin layer has a first hydrophobic layer as a solid layer on the helical spring body and a second hydrophilic layer as a fluid layer arranged on an outer surface of the first hydrophobic layer. A specific gravity of the second hydrophilic layer is smaller than that of the first hydrophobic layer when moistened.

With the second hydrophilic layer (smaller in specific gravity) occupied a larger portion of the synthetic resin layer, it is possible to attain a lightweight guide wire with a high buoyant property insured.

According to other aspect of the invention, the helical spring body has the radiopaque helical portion made of the radiopaque material, a spring-back quantity of which is smaller than that of a stainless steel wire.

Under the effect of the elastic restitution force, the above arrangement makes it possible to reduce the plastic deformation, to which the radiopaque helical portion is subjected although the radiopaque helical portion has the material which is likely to deform plastically.

According to other aspect of the invention, the helical spring body has the radiopaque helical portion made of the radiopaque material, a spring-back quantity of which is smaller than that of a stainless steel wire. The radiotransparent helical portion is made of a stainless steel wire, and an outer diameter of the radiopaque helical portion is smaller than that of the radiotransparent helical portion.

As mentioned above, the elastic restitution force effectuates to reduce the plastic deformation, to which the radiopaque helical portion is subjected although the radiopaque helical portion is liable to deform plastically.

According to other aspect of the invention, the helical spring body is formed by connecting the radiopaque material to the radiotransparent material to produce a wire line element which is lengthwisely stretched to be diametrically thinned and helically wound to serve as a helical coil structure.

Prior to helically winding the radiopaque material and the radiotransparent material, the radiopaque material and the radiotransparent material are linearly connected integrally by means of e.g., a welding procedure and stretched to be diametrically thinned.

This obviates the soldering or brazing procedure to bond the two helical coil portions serially in the prior art structure, thus contributing to produce a lightweight guide wire with a high buoyant performance.

According to other aspect of the invention, the helical spring body has a multitude of line wires, at least one of which contains the radiopaque material at a distal end portion of the helical spring body.

Compared to a single-coiled wire structure in which a single one wire is helically wound to form a general helical spring body, the helical spring body, according to the invention, makes it possible to hold the cylindrical film of the synthetic resin layer at the clearance between the multitude of the line wires without breaking the cylindrical film even when the helical spring body is manipulatively bent excessively to be stretched in a tensile direction. This is because of a very narrow clearance developed between the multitude of the line wires.

According to other aspect of the invention, the floatage chamber encapsulates a foamy body as a discrete foamy structure.

The encapsulated foamy body effectively prevents the elongate core and the helical spring body from being plastically deformed, so as to enhance the elastical restitution force.

According to other aspect of the invention, the floatage chamber encapsulates globular foamy beads, a specific gravity of which ranges from 0.06 to 0.5.

The globular foamy beads decreases a contact area among the neighboring foamy beads, thus creating void areas functionally favorable to forming the floatage chamber so as to contribute to enhance the buoyant performance.

According to other aspect of the invention, there is provided an assembly body of a microcatheter and the medical guide wire, in which an outer diameter of the medical guide wire is approx. 0.2032-0.254 mm (0.008-0.010 inches), and an inner diameter of the microcatheter is approx. 0.280-0.80 mm (0.0110-0.0315 inches).

Although the medical guide wire is diametrically thinned in the assembly body, it is possible to increase the pushability of the medical guide wire with an assist of the microcatheter, thus enabling the operator to insert the medical guide wire deeply into the blood vessel. Consequently, this makes it possible to cope with the demand of the low intrusiveness and lighten the burden, to which the patient owes when therapeutically treated.

According to other aspect of the invention, there is provided an assembly body of a guiding catheter, a balloon catheter and the medical guide wire, in which the medical guide wire is inserted into the guiding catheter while the balloon catheter is guided by the guiding catheter. An outer diameter of the medical guide wire is approx. 0.2032-0.254 mm (0.008-0.010 inches), an inner diameter of the balloon catheter is approx. 0.38-0.90 mm (0.015-0.032 inches) and an inner diameter of the guiding catheter is approx. 1.7-2.0 mm (0.067-0.079 inches).

The assembly body enables the operator to ride the distal end portion on the blood streams with an assist of the buoyance of the floatage chamber and the pressure resistance, so as to deeply insert the medical guide wire into the blood vessel. This makes it possible to diametrically thin the balloon catheter and the guiding catheter respectively, so as to cope with the demand of the low intrusiveness and lighten the burden, to which the patient owes when therapeutically treated.

Preferred embodiments of the present invention are illustrated in the accompanying drawings in which:

Fig. 1 is a side elevational view of a medical guide wire according to a first embodiment of the invention;
Fig. 2 is a latitudinal cross-sectional view taken along the line A-A of Fig. 1;
Fig. 3 is a side elevational view of an elongate core;
Fig. 4 is a plan view of the elongate core;
Fig. 5 is a perspective view of a multi-stepped flat portion;
Figs. 6 and 7 are longitudinal cross sectional views of a distal end portion of the medical guide wire;
Fig. 8 is a schematic view shown when the distal end portion of the medical guide wire is manipulatively bent;
Fig. 9 is a side elevational view shown when the distal end portion of the medical guide wire is pulled to be stretched;
Figs. 10 and 11 are schematic views shown to explain how much a bent portion is expanded when the distal end portion of the medical guide wire is inverted;
Figs. 12 through 15 are photographs shown to observe how a cylindrical film is formed between coil lines of a helical spring portion;
Fig. 16 is an explanatory view shown how the medical guide wire is inserted into the coronary artery;
Fig. 17 is an explanatory view shown how the blood streams reacts to a convex portion of the helical spring portion;
Fig. 18 is a side elevational view of a distal end portion of a medical guide wire according to a second embodiment of the invention;
Fig. 19 is a perspective view of a medical guide wire according to a third embodiment of the invention;
Fig. 20 is a plan view shown how a single-coiled wire structure is manipulatively bent in which a single one wire is helically wound to form a general helical spring body;
Fig. 21 is a plan view shown how a helical spring portion made of a multitude of line wires is manipulatively bent;
Figs. 22 and 23 are longitudinal cross sectional views of a distal end portion of a medical guide wire according to a fourth and fifth embodiment of the invention;
Figs. 24 and 25 are schematic views shown to explain how a medical guide wire is inserted into a highly occluded area with an assist of an assembly body of a medical guide wire and a microcatheter according to a sixth embodiment of the invention;

Fig. 26 is a side elevational view of an elongate core according to a modification form of the invention;

Fig. 27 is a right elevational view of the elongate core according to the modification form of the invention;

Fig. 28 is a perspective view of a multi-stepped tip portion according to the modification form of the invention;

Fig. 29 is a side elevational view of a medical guide wire according to another modification form of the invention;

Fig. 30 is a latitudinal cross sectional view taken along the line B-B of Fig. 29;

Fig. 31 is a latitudinal cross sectional view taken along the line C-C of Fig. 29; and

Fig. 32 is a side elevational view of a medical guide wire according to yet another modification form of the invention.

To describe the embodiments in a more specific manner, structures of a medical guide wire and a helical spring body are explained in detail by referring to the accompanied drawings.

In the following description of the depicted embodiments, the same reference numerals are used for features of the same type.

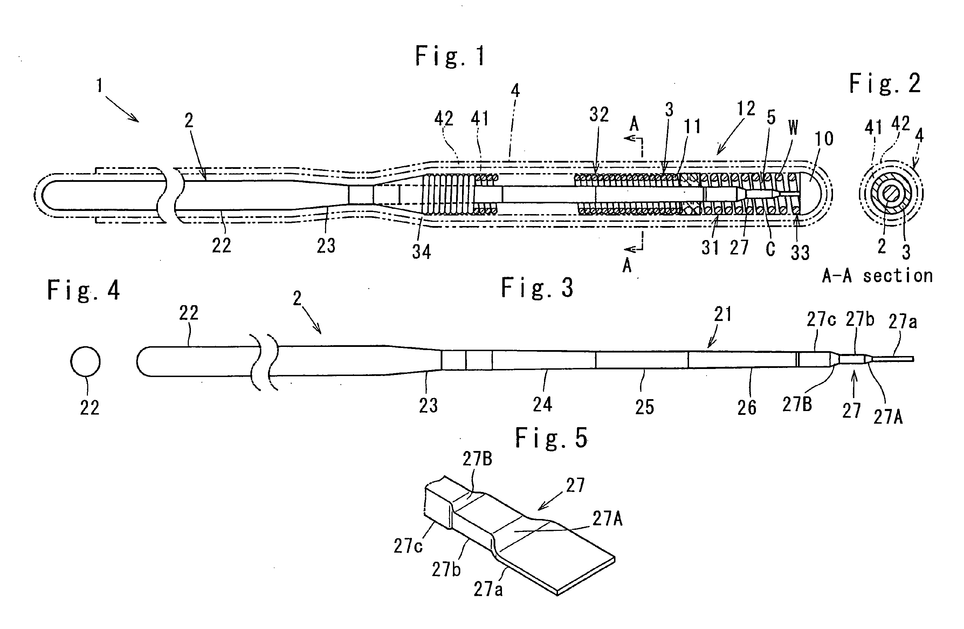

Referring to Figs. 1-17 which structurally show a medical guide wire 1 according to a first embodiment of the invention, the medical guide wire 1 is used to therapeutically treat an occluded area of the coronary artery.

In this situation, the right side of the drawings means a distal end side of the medical guide wire 1, and the left side of the drawings means a proximal end side (rear end side) of the medical guide wire 1, unless specified otherwise hereinafter.

The medical guide wire 1 has an elongate core 2 and a helical spring body 3 in which a distal end portion 21 of the elongate core 2 is concentrically inserted as shown in Fig. 1. On an outer surface of the helical spring body 3, a synthetic resin layer 4 is coated. A floatage chamber 5 is provided within a distal end portion 12 of the medical guide wire 1.

The elongate core 2 is made of a stainless steel wire as shown in Figs. 2-4. The elongate core 2 has the distal end portion 21 diametrically thinned, and having a proximal end portion 22 diametrically thickened. The distal end portion 21 measures approx. 300 mm in length, and the proximal end portion 22 (i.e., the rest of the elongate core) measures approx. 1200 mm or 2700 mm in length.

From a proximal side to a distal side of the elongate core 2, the distal end portion 21 consecutively has an acutely tapered portion 23, a moderately tapered portion 24, a columnar portion 25, a slightly tapered portion 26 and a multi-stepped flat portion 27 as shown in Fig. 5.

The multi-stepped flat portion 27 has a first segment 27a, a second segment 27b and a third segment 27c through stepped portions 27A, 27B from the distal side to the proximal side. The first segment 27a, which resides at a foremost head of the elongate core 2, has a thickness smaller than any of the segments 27b, 27c. The second segment 27b, which follows the first segment 27a in the immediate rear, has a thickness slightly greater than the first segment 27a. The third segment 27c, which follows the second segment 27b in the immediate rear, has a thickness slightly greater than the second segment 27b.

When the multi-stepped flat portion 27 is manipulatively bent, the segments 27a, 27b, 27c represent their radius of curvature stepwisely increasing in this order.

The multi-stepped flat portion 27 makes it possible to curvedly deform the distal end portion 21 within a narrow range, thus enabling the operator to staunchly follow the distal end portion 21 along a sinuous path of the vascular stricture area upon inserting the distal end portion 21 into the blood vessel.

By way of illustration, the thickness of the segments 27a, 27b, 27c in turn measures 0.040 mm, 0.050 mm and 0. 063 mm.

In the case in which each latitudinal cross sectional area of the segments 27a, 27b, 27c is substantially uniform through its lengthwise direction, the elongate core 2 substantially aligns the distal end portion 21 in parallel with a press mould die when pressing the distal end portion 21 between an upper and lower press block (not shown). This makes minute dimensions of the segments 27a, 27b, 27c stable with higher precision, and lengthens a service life of the press mould die.

The helical spring body 3 is formed by connecting a platinum wire to a stainless steel wire to produce a linear wire element which is lengthwisely stretched to be diametrically thinned and helically wound to serve as a helical coil structure. An entire length of the helical spring body 3 is approx. 300 mm, a dimension of which is substantially the same as that of the distal end portion 21 of the elongate core 2.

A front side of the helical spring body 3 has a radiopaque helical portion 31 (approx. 30 mm in length) made of a platinum or the like, and a rear side of the helical spring body 3 has a radiotransparent helical portion 32 (approx. 270 mm in length) made of a stainless steel or the like.

Instead of the platinum wire applied to the helical spring body 3 as a radiopaque material, a gold wire, a silver wire or a tungsten wire (wolfram wire) may be used. As a radiotransparent material, the stainless steel wire has been used from the biocompatible point of view.

The radiopaque material such as the platinum wire is liable to plastically deform with a spring-back quantity smaller than the stainless steel wire.

By forming the helical spring body 3 by line wires (0.072 mm in outer diameter) which are wound to be the helical coil structure (0.355 mm in outer diameter), an outer diameter of the radiopaque helical portion 31 becomes smaller by over 0.02 mm than that of the radiotransparent helical portion 32. This means that the helical spring body 3 is diametrically reduced progressively (tapered off) as approaching forward. The radiopaque helical portion 31 has a clearance C between the coil lines W of the helical spring body 3. A width of the clearance C is approx. 10-30 % of an outer diameter of the coil lines W to impart the radiopaque helical portion 31 with a good flexibility.

A distal end 33 of the helical spring body 3 and a distal end of the elongate core 2 are air-tightly bonded at a securement portion (soldering portion) 10 without any gap by the use of spherical tin grains (tin pellets), a brazing alloy or the like. A proximal end 34 of the helical spring body 3 is firmly fixed to the acutely tapered portion 23 by means of the soldering procedure. The synthetic resin layer 4 extends from the outer surface of the helical spring body 3 to the proximal side portion 22 of the elongate core 2.

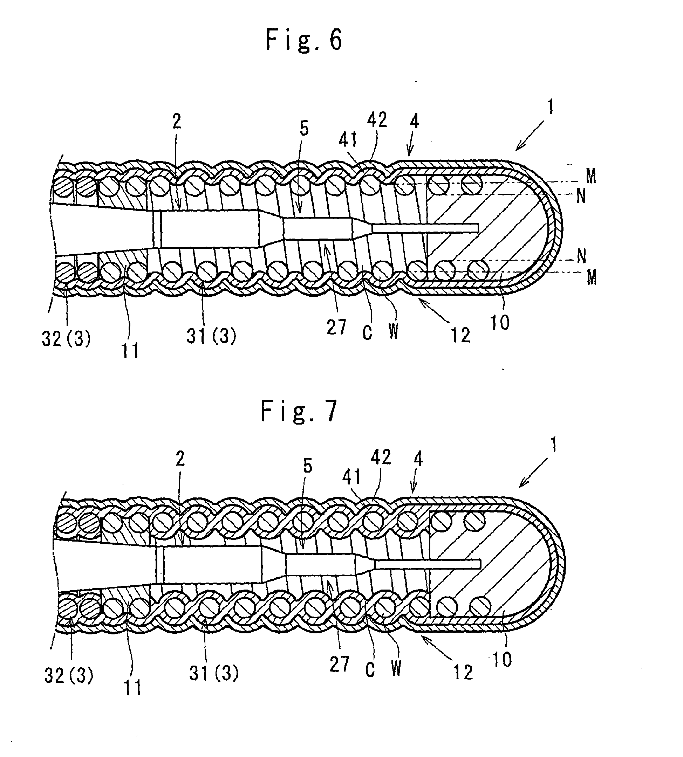

The synthetic resin layer 4 has a first hydrophobic layer (solid layer) 41 and a second hydrophilic layer (viscous fluid layer) 42 placed over the first hydrophobic layer 41 as shown at a double-layered structure in Figs. 6, 7.

The second hydrophilic layer 42 exhibits a lubricious property when it is moistened. The first hydrophobic layer 41 is formed as a hydrophobic coating by polyurethane, polyether block amide, polyethylene, polyamide, fluoric polymer or the like. The second hydrophilic layer 42 is formed as a hydrophilic coating by polyvinylpyrrolidone, maleic anhydride ethylester copolymer, polyethylene oxide or the like.

The specific gravity of the second hydrophilic layer 42 becomes smaller than that of the first hydrophobic layer 41 when moistened.

When the first hydrophobic layer 41 is formed with PTFE (poly-tetra-fluoro-ethylene, specific gravity: 2.14-2. 20), polyvinylpyrrolidone, maleic anhydride ethylester copolymer or polyethylene oxide (specific gravity: substantially near to that of water) is applied to the second hydrophilic layer 42. When the first hydrophobic layer 41 is formed with polyurethane (specific gravity: 1.20-1.24) or polyether (specific gravity: 1.38), polyvinylpyrrolidone (specific gravity: substantially near to that of water) may be used to the second hydrophilic layer 42.

The first hydrophobic layer 41 and the second hydrophilic layer 42 may be in turn coated on the outer surface of the helical spring body 3 with the first hydrophobic layer 41 as an inner coating layer as shown in Fig. 6.

Alternatively, the first hydrophobic layer 41 may be coated on an entire surface of the coil lines W before coating the second hydrophilic layer 42 over the first hydrophobic layer 41 as shown in Fig. 7.

At a connection portion between the radiopaque helical portion 31 and the radiotransparent helical portion 32, a hermetical wall 11 is provided by means of the soldering procedure. The hermetical wall 11 air-tightly bonds a proximal end of the radiopaque helical portion 31 to the elongate core 2 with no gap appeared by means of the spherical tin grains (tin pellets), the brazing alloy or the like.

The hermetical wall 11 together with the securement portion 10 and the synthetic resin layer 4 hermetically defines an interior space within the radiopaque helical portion 31 so as to form the floatage chamber 5.

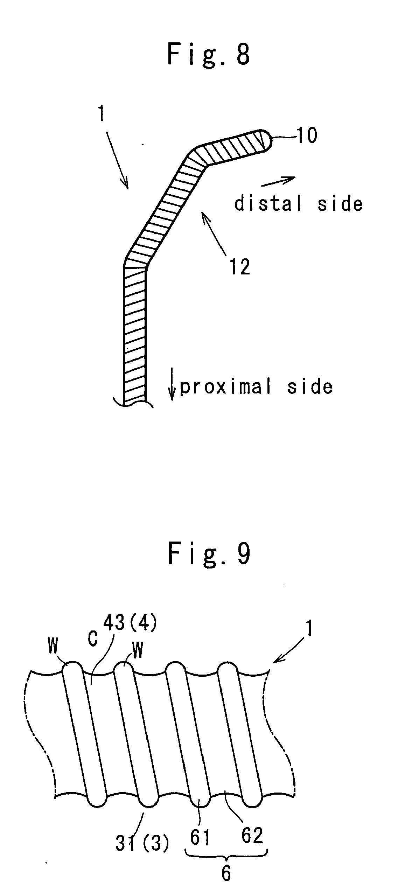

When the helical spring body 3 is expanded to broaden the clearance C upon manipulatively bending the medical guide wire 1, the bending force stretches the synthetic resin layer 4 to form a cylindrical film 43 at the clearance C as shown in Fig. 9. The stretched film 43 decreases its outer diameter smaller than that of the helical spring body 3, and maintain an inner space air-tight within the helical spring body 3.

As shown in Fig. 8, the radiopaque helical portion 31 is preshaped at one or more places within the range of approx. 2-7 mm from the distal end of the radiopaque helical portion 31, so as to enable the operator to selective insertions against the branched portion of the blood vessel.

Upon inserting the helical spring body 3 into the vascular stricture area, the insertion force elastically deforms the helical spring body 3 due to the contact with the vascular wall of the blood vessel.

In this situation, the helical spring body 3 is stretched to widen the clearance C as shown in Fig. 9. The synthetic resin layer 4, however, absorbes the aquatic component (water) to be moistened, and stretches to consecutively form the cylindrical film 43 along the clearance C.

The film 43 is in the form of a spiral tube configuration. The film 43 has a concave configuration, an outer diameter of which is smaller than that of the synthetic resin layer 4 coated on the outer surface of the helical spring body 3.

Namely, an outer surface of the synthetic resin layer 4 undulates to form a concave-convex portion 6 with a convex portion 61 placed at the coil lines W and a concave portion 62 at the clearance C in which the cylindrical film 43 stretches inward.

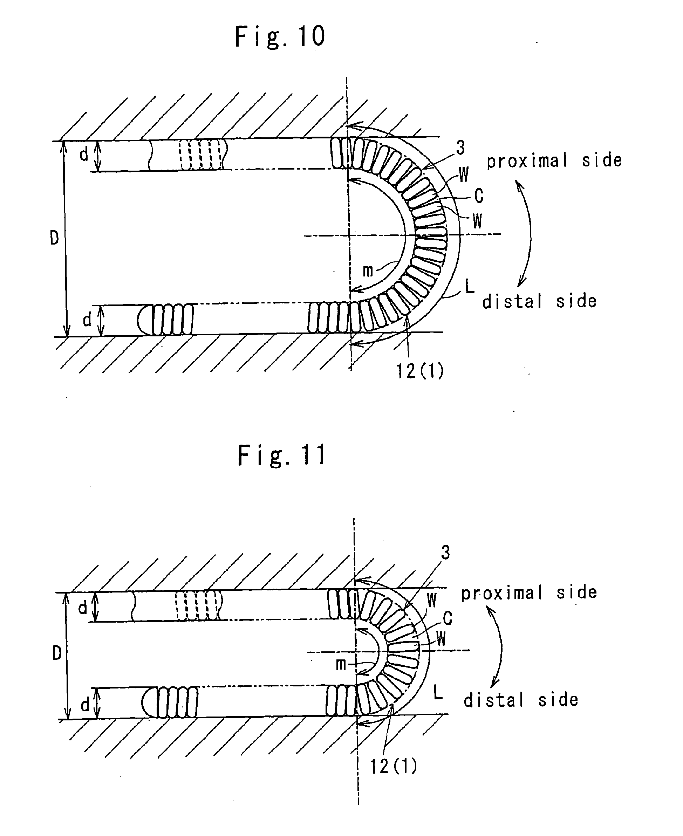

In general, the coronary artery has an inner diameter around 2-4 mm and becomes thinner as approaching inward. Upon inserting the helical spring body 3 into the coronary artery as shown in Figs. 10, 11, the helical spring body 3 inverts its distal end portion at the vascular stricture area however seldom if ever.

By way of illustration, when an outer diameter (d) of the medical guide wire is 0.35 mm as shown in Fig. 10, and an inner diameter (D) of the coronary artery is 2.0 mm, a difference ($\Delta$s) between an outer arcuate length (L) and an inner arcuate length (m) of the helical spring body 3 is determined at a bent portion as follows:

$$\Delta s = L - m$$

$$= (\pi \times 2.0 \times 1/2) - (\pi \times 1.3 \times 1/2) \square 1.099 \, mm$$

This means that the inner arcuate length (m: 2.04 mm) becomes to 3.139 mm so as to yield an elongation ratio as 1.53. This signifies that the helical spring body 3 is resultantly stretched by approx. 50 %.

When the inner diameter (D) of the coronary artery is 1.4 mm as shown in Fig. 11, the difference (Δs) is determined at the bent portion as follows:

$$\Delta s = L - m$$

$$= (\pi \times 1.4 \times 1/2) - (\pi \times 0.7 \times 1/2) \square 1.099 \, mm$$

This means that the inner arcuate length (m:1.099 mm) becomes to 2.198 mm so as to yield the elongation ratio as 2.0. This signifies that the helical spring body 3 is resultantly stretched by approx. 100 %. When the inner diameter (D) of the coronary artery is less than 1.4 mm exclusive, the above phenomenon will not occur because the helical spring body 3 is contained within the coronary artery due to its limited diametrical width.

The cylindrical film 43 is determined not to be broken by the selection of its material and quantity even when the film 43 is stretched by 50 % or 100 % at the time when the helical spring body 3 is inverted as the bent portion within the minute blood vessel.

In more specific manner, a plasticizer may be added to the hydrophobic polymer to increase the flexibility of the first hydrophobic layer 41. As the plasticizer, used are camphor, castor oil, dioctylphthalate or the like.

Upon forming the synthetic resin layer 4, used are an extrusion method, dipping method or method of coating a heat-shrinkage tube. Any method can be applied as long as it can air-tightly seal the floatage chamber 5.

In order to hermetically seal the floatage chamber 5, it is desirable to use the dipping method or the method of coating the heat-shrinkage tube. This is because the desirable method forms the synthetic resin layer 4 without permitting the synthetic resin to enter into the floatage chamber 5, although the heat-shrinkage tube permits the floatage chamber 5 to be pressed, while keeping the gaseous component in the floatage chamber 5.

Among the above methods, the dipping method is the most desirable one since it can obviate the necessity of trimming the end of the synthetic resin layer without permitting the floatage chamber 5 to be pressed.

Upon forming the synthetic resin layer 4, the helical spring body 3 is dipped (dipping step) into the hydrophobic polymer solution and desiccated thereafter at the temperature of approx. 170 □ for about ten minutes. After coating the hydrophobic polymer on the helical spring body 3, the helical spring body 3 is dipped (finish dipping) into the hydrophilic polymer solution and desiccated thereafter at the temperature of approx. 170 □ for about ten minutes.

To the hydrophobic polymer solution, an adhesive polymer, the plasticizer, or both of them may be added to form a solution mixture. After the helical spring body 3 is dipped into the solution mixture and desiccated, the helical spring body 3 may be dipped into the hydrophilic polymer solution, otherwise the hydrophilic polymer may be coated on the hydrophobic polymer of the helical spring body 3.

As the adhesive polymer, used are polyurethane, polyester, styrenepolybutadiene, acrylic resin or the like from the fact that these polymers advantageously strengthen an adherence of the synthetic resin layer 4 to the helical spring body 3.

As the plasticizer, used are camphor, castor oil, dioctylphthalate or the like from the reason that these polymers are beneficial to enhancing the flexibility of the synthetic resin layer 4.

It is particularly preferable to add the plasticizer to provide a good flexibility so as not to break the cylindrical film 43 consecutively formed along the clearance C of the coil lines W when the helical spring body 3 is inverted as aforementioned.

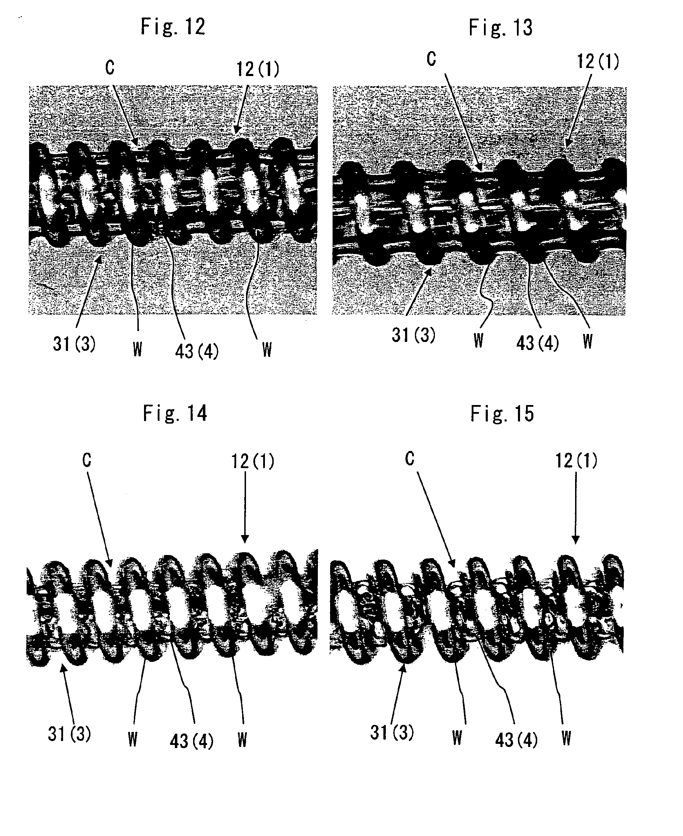

Figs. 12 through 15 are photographs of the cylindrical film 43 observed along the clearance C between the coil lines W of the helical spring body 3.

In Figs. 12, 13, the cylindrical film 43 is located at a central diameter portion M of the helical spring body 3 when stretched in turn by approx. 50% and 100%. The central diameter portion M is an average diameter of an inner and outer diameter of the helical spring body 3 as shown for the purpose of convenience in Fig. 6.

In Figs. 14, 15, the cylindrical film 43 is located at an underside N of the helical spring body 3 when stretched in turn by approx. 50% and 100%. The underside N means an inner surface of the helical spring body 3 as shown for the purpose of convenience in Fig. 6.

In each case, the cylindrical film 43 resides at the clearance C between the coil lines W and contains the gaseous component inside the helical spring body 3.

Namely, even when the medical guide wire 1 bends the radiopaque helical portion 31 of the helical spring body 3 to broaden the clearance C between the coil lines W, it is possible to contain the gaseous component inside the helical spring body 3 without releasing it outside, so as to maintain the air-tightness within the helical spring body 3 to keep the floatage chamber 5 in shape. This is due to the cylindrical film 43 formed by stretching the synthetic resin layer 4 in the moistened condition when the helical spring body 3 is manipulatively bent upon inserting the helical spring body 3 into the blood vessel.

According the invention, by defining the floatage chamber 5 with the cylindrical film 43, the following advantages are obtained:

(a) With the floatage chamber 5 formed within the helical spring body 3, it is possible to maintain the stable posture of the distal end portion 12 in the blood streams. The platinum wire is applied at least to the radiopaque helical portion 31 as a contrast medium for the fluoroscopy. The specific gravity of the platinum wire is 21.4 which is approx. 2.7 times as great as that of the stainless steel wire (specific gravity: 7.9).

The elongate core 2 is diametrically thinned to cope with the demand to maintain the distal end portion 12 pliable. In this situation, the medical guide wire 1 is liable to significantly hang down the distal end portion 12 in the unrestricted state as the radiopaque helical portion 31 increases its specific gravity. This holds true when inserting the distal end portion 12 into the blood vessel.

When the medical guide wire 1 hangs down the distal end portion 12 upon inserting the radiopaque helical portion 31 into the blood vessel, the distal end portion 12 increases chances of contact with the vascular wall of the blood vessel, so as to invite the vascular disassociation or the separation of the intima. In particular, the sagging of the distal end portion 12 reduces the selectiveness at the branched portion of the blood vessel when navigating the distal end portion 12 in the desired direction.

Contrary to the above situation, according to the invention, the floatage chamber 5 is provided within the radiopaque helical portion 31 so as to reduce the sagging of the distal end portion 12 in the blood streams due to the buoyance of the floatage chamber 5. This enables the operator to keep the distal end portion 12 substantially in the linear straight condition upon navigating the medical guide wire 1 in the blood vessel.

By reducing the sagging of the distal end portion 12 in the blood streams, it is possible to significantly mitigate the contact and friction of the distal end portion 12 against the vascular wall of the blood vessel. This enables the operator to navigate the distal end portion 12 so as to deeply insert it smoothly into the sinuous and meandrous blood vessel without inviting the vascular disassociation or the separation of the intima.

(b) With the elastical restitution force derived from the floatage chamber 5, it is possible to stably keep the distal end portion 12 in shape, so as to enable the operator to a deep insertion of the distal end portion 12 into the blood vessel.

Since the spring-back quantity of the radiopaque helical portion 31 is smaller than that of the radiotransparent helical portion 32, and radiopaque helical portion 31 plastically deforms more easily than the radiotransparent helical portion 32. This renders the distal end portion 12 to readily deform and liable to manipulatively bend.

In this situation, the medical guide wire 1 has the floatage chamber 5 hermetically inside the radiopaque helical portion 31.

With the floatage chamber 5 placed inside the radiopaque helical portion 31, upon manipulatively bending the radiopaque helical portion 31 into a bent position, the bending force increases a pneumatic pressure within the floatage chamber 5, upon releasing the bending force, the increased pneumatic pressure returns the radiopaque helical portion 31 from the bent position to the original position.

Namely, with the use of the elastical restitution force developed by filling the floatage chamber 5 with the gaseous component, it is possible to reduce the plastic deformation of the distal end portion 12 so as to always maintain an initial good shape of the distal end portion 12 stably.

Due to the spring-back quantity difference between the radiopaque helical portion 31 (e.g., platinum wire) and the radiotransparent helical portion 32 (e.g., stainless steel wire), the spring-back force renders the helical spring body 3 to decrease its outer diameter progressively as approaching the distal end 33 of the radiopaque helical portion 31 (tapered off).

This makes it possible to enhance the passage of the radiopaque helical portion 31 through the vascular stricture area all the more with the effect of maintaining the initial good shape of the distal end portion 12.

(c) A pressure resistance enables the operator to deeply insert the helical spring body 3 into the blood vessel as described hereinafter in detail.

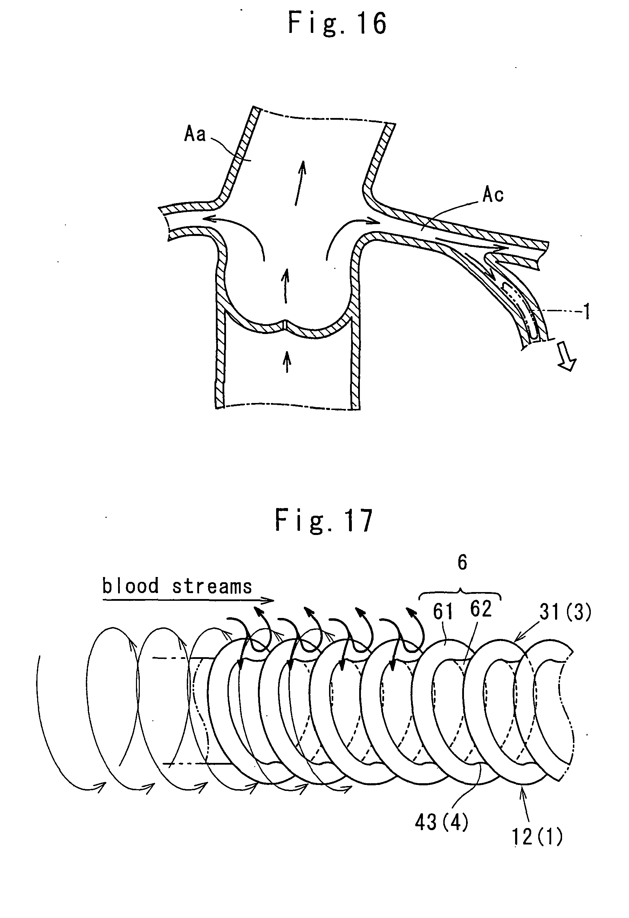

The medical guide wire 1, according to the invention, is used for therapeutically treating the occluded area of the coronary artery, and inserted into the coronary artery Ac as shown in Fig. 16.

As designated at arrows in Fig. 16, contrary to the case of the aortic arch Aa, the blood in the coronary artery Ac flows in the same direction as the medical guide wire 1 navigates the distal end portion 12 along the blood vessel, as it were a tail wind situation.

In this instance, the helical spring body 3 permits the blood streams to collide against the convex portion 61, while intercepting an entry of the blood streams toward the elongate core 2. This makes the blood streams flow along the coil-winding configuration of the helical spring body 3 so as to develop spiral currents as shown in Fig. 17.

This instance produces a forward propelling force for the helical spring body 3 due to the spiral currents together with the pressure resistance developed when the blood streams collide against the convex portion 61.

With the floatage chamber 5 provided to contain the gaseous component inside the floatage chamber 5, it is possible to lift the distal end portion 12 in the blood streams due to the buoyance of the floatage chamber 5, while at the same time, insuring the forward propelling force derived from the spiral currents and the pressure resistance. This enables the operator to readily insert the helical spring body 3 deeply into the blood vessel.

(d) With the floatage chamber 5 formed to provide the buoyance, it is possible to diametrically reduce (thin) the medical guide wire 1, thus coping with the demand of low intrusiveness against the patient so as to lighten the burden, to which the patient owes.

Upon therapeutically dilating the occluded area of cardiovascular system, i.e., implementing the percutaneous transluminal coronary angioplasty (PTCA) by way of example, generally used are a medical guide wire (0.35 mm in outer diameter) and a guiding catheter (7F-8F: 2.3-2.7 mm in inner diameter) which introduces a balloon catheter to implement the vascular dilatation. The medical guide wire, in general, has 0.355 mm in outer diameter to satisfy various mechanical properties such as torque-transmissibility and pushability needed to advance the helical spring body 3 into the tortuous and meandrous blood vessel.

In addition to the above mechanical properties needed to enhance the deep insertion into the blood vessel, the medical guide wire 1 has the capability to ride on the blood streams due to the buoyance of the floatage chamber 5. This mitigates the mechanical properties to diametrically reduce the medical guide wire 1 and the elongate core 2 so as to produce a thinned guide wire.

Upon using as an assembly body (not shown) of the medical guide wire 1, the balloon catheter and the guiding catheter, the buoyance renders to reduce an outer diameter of the medical guide wire 1 from 0.014 inches (0.355 mm) to 0.008-0.010 inches (0.2032-0.254 mm), while decreasing an inner diameter of the guiding catheter from 7F-8F (2.3-2.7 mm) to 5F-6F (1.7-2.0 mm). This makes it possible to cope with the demand of the low intrusiveness against the patient so as to lighten the burden, to which the patient owes at the time of therapeutically operating the medical guide wire 1.

The balloon catheter usually has an inner diameter around 0.38-0.90 mm, and the guiding catheter is used together with the balloon catheter. The guiding catheter maintains an appropriate annular space with the medical guide wire 1. By sharing the pushing force of the medical guide wire 1 with the guiding catheter and the balloon catheter as a reactionary force, it is possible for the medical guide wire 1 to exert its pushability against the diseased area.

(e) With the helical spring body 3 hermetically sealed by the synthetic resin layer 4, even when the medical guide wire 1 locally buckles the distal end portion 12, the buckling force increases the pneumatic pressure inside the floatage chamber 5, so as to develop a restitution force necessary to restore the buckled portion due to the elastical recovery. The synthetic resin layer 4 also serves to protect the elongate core 2 which is likely to plastically deform.

(f) Due to the double-layered structure defined by the first hydrophobic layer (solid layer) 41 and the second hydrophilic layer (viscous fluid layer) 42, even if minute pores (pinholes) or injuries may be developed on the synthetic resin layer 4, it is possible to avoid the gaseous leakage from the floatage chamber 5, so as to hermetically maintain the floatage chamber 5 by covering an entire surface of the first hydrophobic layer 41 with the viscous fluid of the second hydrophilic layer 42.

By covering the first hydrophobic layer 41 with the viscous fluid of the second hydrophilic layer 42, it is possible to lessen the friction of the synthetic resin layer 4 against the vascular wall in the blood vessel.

(g) By determining the specific gravity of the second hydrophilic layer 42 to be smaller than that of the first hydrophobic layer 41, it becomes possible to make the medical guide wire 1 lightweight with a higher buoyant performance.

This is because the second hydrophilic layer 42 increases its diametrical dimension from the inside to the outside more than the first hydrophobic layer 41 increases its diametrical dimension, so that the second hydrophilic layer 42 largely occupies the synthetic resin layer 4 in weight when the two layers 41, 42 have an identical thickness.

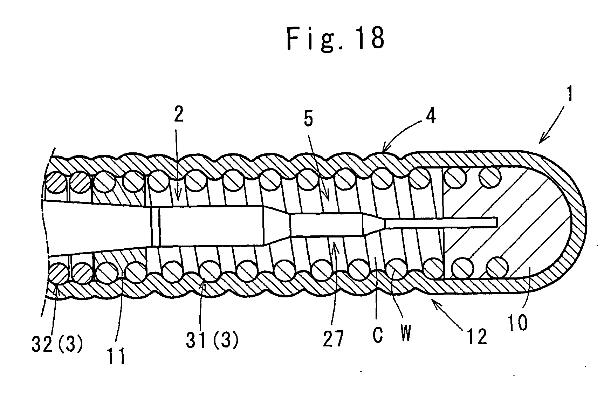

Fig. 18 shows a second embodiment of the invention in which described are component parts only different from the structure specified by the first embodiment of the invention.

The synthetic resin layer 4, according to the second embodiment of the invention, is a mixture of the hydrophobic polymer and the hydrophilic polymer to form one single layer structure. The synthetic resin layer 4 decreases its specific gravity from the inner side to the outer side of the one single layer structure in the moistened condition.

Instead of the mixture formed by the hydrophobic polymer and the hydrophilic polymer, the hydrophilic polymer may be added to a mixture of the hydrophobic polymer and the adhesive polymer upon forming the synthetic resin layer 4. Otherwise, the hydrophilic polymer may be mixed to a mixture of the hydrophobic polymer and the plasticizer. Alternatively, the hydrophilic polymer may be mixed to the mixture of the hydrophobic polymer, the adhesive polymer and the plasticizer.

As the hydrophobic polymer, used are cellulose ester, or copolymer of polymethylvynlether, maleic anhydrite and like. Among these polymers, cellulose ester is the most preferable.

As the hydrophilic polymer, used are polyvinylpyrrolidone, maleic anhydride ethylester copolymer, polyethylene oxide and the like.

As the adhesive polymer, used are polyurethane, polyester, styrenepolybutadiene, acrylic resin and the like from the fact that these polymers advantageously strengthen an adherence of the synthetic resin layer 4 to the helical spring body 3.

As the plasticizer, used are camphor, castor oil, dioctylphthalate and the like from the reason that these polymers are effective in enhancing the flexibility of the synthetic resin layer 4.

According to the method of forming the synthetic resin layer 4, the helical spring body 3 is dipped (dipping step) into the mixture of the hydrophobic polymer solution and the hydrophilic polymer solution, and desiccated thereafter at the temperature of approx. 170□ for about ten minutes.

Alternatively, to the hydrophobic polymer solution, the adhesive polymer or the plasticizer, or both of them may be added so as to form a solution mixture. After the helical spring body 3 is dipped into the solution mixture, and desiccated at the temperature of approx. 170 □ for about ten minutes.

The synthetic resin layer 4, according to the second embodiment of the invention, decreases its specific gravity from the inner side to the outer side of the one single layer structure in the moistened condition.

To the outer surface of the helical spring body 3, the mixture of the hydrophobic polymer and the adhesive polymer is adhered. The hydrophobic polymer and the hydrophilic polymer are bonded by high bridging force or intermolecular force. For this reason, the hydrophobic polymer component resides more at the underside of the helical spring body 3, and the hydrophilic polymer component resides more at the outerside of the helical spring body 3.

The above arrangement makes it possible to make the medical guide wire 1 light weight so as to contribute to a higher buoyant performance.

This is because the outer component of the synthetic resin layer 4 increases its diametrical dimension progressively more than the inner component of the synthetic resin layer 4 increases its diametrical dimension, so that the outer component largely occupies the synthetic resin layer 4 in weight with the specific gravity of the outer component determined to be smaller.

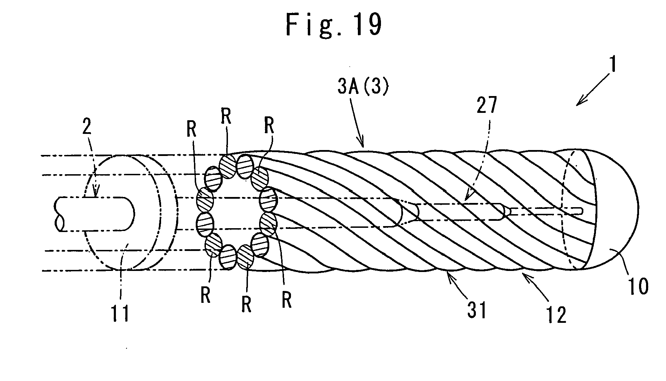

Figs. 19 through 21 show a third embodiment of the invention in which the radiopaque helical portion 31 forms a multiple helical wire structure 3A. The multiple helical wire structure 3A has a multitude of coiled wires, at least one of which is made of the radiopaque material.

In more specific terms, upon forming the multiple helical wire structure 3A, linear wires ranging from three to twelve pieces (e.g., 12 pieces shown in Fig. 19) are used, each outer diameter ($\varphi$) of which measures 0.072 mm. Thereafter, the linear wires are helically twisted to form a wire-stranded structure, an outer diameter of which measures 0.35 mm.

Namely, between the securement portion 10 and the hermetic wall 11, the radiopaque helical portion 31 is formed as the radiopaque helical portion by alternately stranding radiopaque wires R and the other linear wires. In this instance, the number of the radiopaque wires R and the number of the other linear wires are counted as six respectively.

As a method of forming the radiopaque helical portion 31, introduced is one that the linear wires are wound around a mandrel core (not shown), or the linear wires are helically twisted with the use of a rope-stranding machine. Alternatively, three or four pieces of the linear wires ($\varphi$0.072 mm) are helically twisted to form a stranded wire unit beforehand, and then the units ranging from three to twelve may be stranded to prepare the multiple helical wire structure 3A.

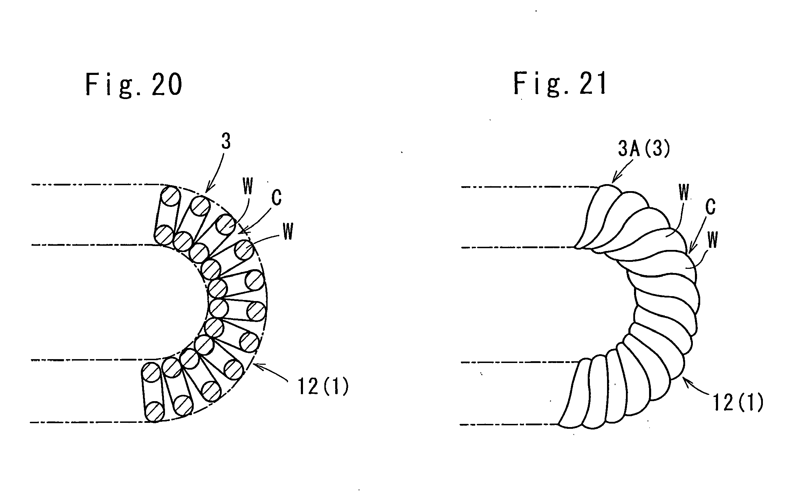

Such is the structure that when the helical spring body 3 is manipulatively bent, the bending force makes the clearance C extremely small at an outer side of the bent portion in which the helical spring body 3 (multiple helical wire structure 3A) exhibits a larger radius of curvature as shown in Fig. 21, contrary to the broadened clearance C which the helical spring body 3 (single-coiled wire structure) exhibits as shown in Fig. 20.

This is because, at the time of bending the helical spring body 3, the bending force develops the minute positional slips between the coil lines of the multiple helical wire structure 3A so as to reduce the clearance C therebetween.

Due to the reduced clearance C in the multiple helical wire structure 3A, it is possible to prevent the synthetic resin layer 4 from being broken without being intolerably stretched even when the multiple helical wire structure 3A is excessively bent, thus maintaining the air-tightness inside the multiple helical wire structure 3A, so as to stably keep the normal function of the floatage chamber 5 for an extended period of time.

The above comparison consequently shows how advantageously the multiple helical wire structure 3A relates the synthetic resin layer 4 to the floatage chamber 5.

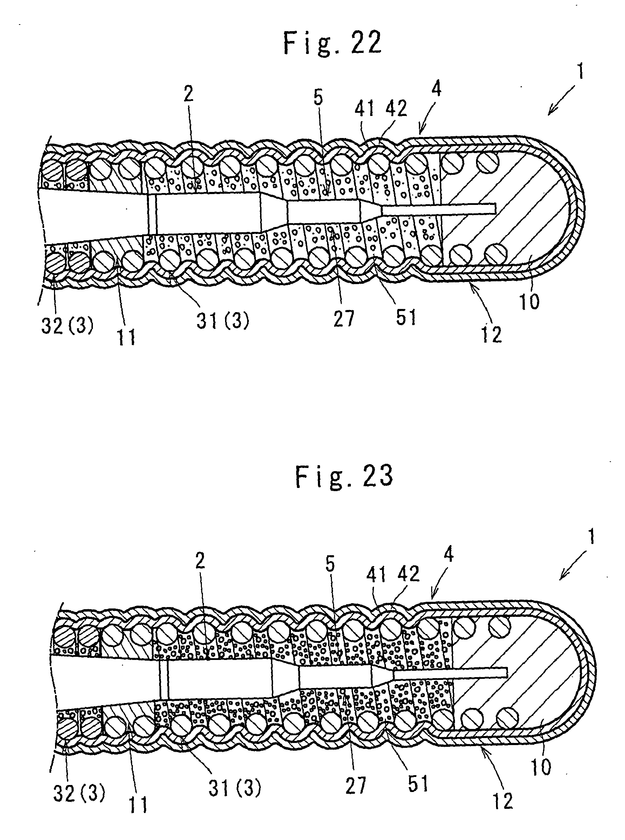

Fig. 22 shows a fourth embodiment of the invention in which the floatage chamber 5 is formed as a plenum portion by a foamy body layer 51 in the shape of a discrete foamy structure.

Upon providing the foamy body layer 51, after the helical spring body 3 is fixedly secured to the elongate core 2 by means of the soldering procedure, the helical spring body 3 is dipped to a certain depth into a bath which contains foamy liquid material, so as to form the foamy body layer 51 inside floatage chamber 5.

After lifting the helical spring body 3 from the bath, the helical spring body 3 is wrought through a special jig (not shown) so as to trim the outer diameter of the foamy body layer 51.

Thereafter, the helical spring body 3 is left as it is, or heated until the foamy body layer 51 is desiccated. Then, the synthetic resin layer 4 is provided on the outer surface of helical spring body 3 by means of the dipping procedure. Instead of the dipping procedure, a spray type of foamy liquid may be used.

The foamy body layer 51 is provided by adding a foamy agent to a synthetic resin. The synthetic resin represents polyester, copolymer of styrene and methacrylic acid(styrene-based resin) and polyethylene, polypropylene (polyolefin-based resin).

In the meanwhile, the foaming agent represents carbon dioxide (volatile foamy agent) and ammonium carbonate (degradable foamy agent). By way of example, a bridged-bond type polyolefin foamy agent (specific gravity: 0.06-0.3) may be used.

The foamy body layer 51 may be formed by adding the foamy agent to a rubber (silicone rubber, chloroprene rubber). The foamy agent for the silicone rubber represents azobisisobutylnitrile as a well-known substance. As for the foamy body layer 51, a texture in which foams are discretely arranged is preferable to a texture in which foams are continuously arranged. The minute foams contained in the foamy body layer 51 are preferable. A silicone sponge (minute cell-texture and 110 $\mu$m on average cellular diameter) is preferable which has a low specific gravity (approx. 0.41) with an excellent permanent strain exhibited when a contractile stress is applied.

Under the presence of the foamy body layer 51 between the elongate core 2 and the helical spring body 3, it is possible to prevent the synthetic resin from invading into the floatage chamber 5 even if the helical spring 3 is depressed upon applying the synthetic resin layer 4 to the outer surface of the helical spring body 3 by means of an extrusion procedure.

With the foamy body layer 51 formed in the shape of the discrete foamy structure, it is possible to effectively avoid the synthetic resin from invading into the floatage chamber 5 upon applying the synthetic resin layer 4 to the outer surface of the helical spring body 3.

Due to the foamy body layer 51 being of an elastical material, it effectively prevents the elongate core 2 and the helical spring body 3 from being plastically deformed, so as to strengthen a restitutive force developed upon manipulatively bending the distal end portion 12 of the medical guide wire 1.

In order to provide the radiopaque helical portion 31 with the flexibility, it is arranged to develop a tiny clearance between the coil lines of the radiopaque helical portion 31. If the synthetic resin invades into the tiny clearance between the coil lines upon forming the synthetic resin layer 4, it would hinder the good flexibility of the radiopaque helical portion 31.

With the floatage chamber 5 formed by the foamy body layer 51, it becomes possible to extend the foamy body layer 51 over the outer surface of the radiopaque helical portion 31, thus stably maintaining the good flexibility of the radiopaque helical portion 31.

Fig. 23 shows a fifth embodiment of the invention in which the floatage chamber 5 is formed as a plenum portion with globular grains 53 (synthetic foamy beads, microballoons). The material for the globular grains 53 (e.g., approx. 0.06-0.5 and 50-100 $\mu$m in terms of the specific gravity and granular size) is selected from the chemical substance described in the fourth embodiment of the invention.

With the foamy beads or microballoons having less chances to come in contact with the neighboring beads or microballoons contrary to a polygonal structure, it is possible to insure larger spatial void portions among the beads, which is functionally favorable to the floatage chamber 5.

By using the foamy body layer 51 as a binder for the globular grains 53, it is possible to readily form the floatage chamber 5 within the radiopaque helical portion 31, while at the same time, increasing the buoyance by containing the lightweight gas in the floatage chamber 5.

In this instance, upon forming the floatage chamber 5, the same method can be used as mentioned at the fourth embodiment of the invention except for the process in which a certain amount of the globular grains 53 is added to the foamy body layer 51.

With the use of inorganic microballoons, it is possible to increase the contractile strength, to which the distal end portion 12 of the medical guide wire 1 is subjected upon manipulatively bending the medical guide wire 1.

This can be done without leaking gaseous component out of the floatage chamber 5. Lightweight gas (e.g., helium) may be contained in the floatage chamber 5 to increase the buoyance of the floatage chamber 5.

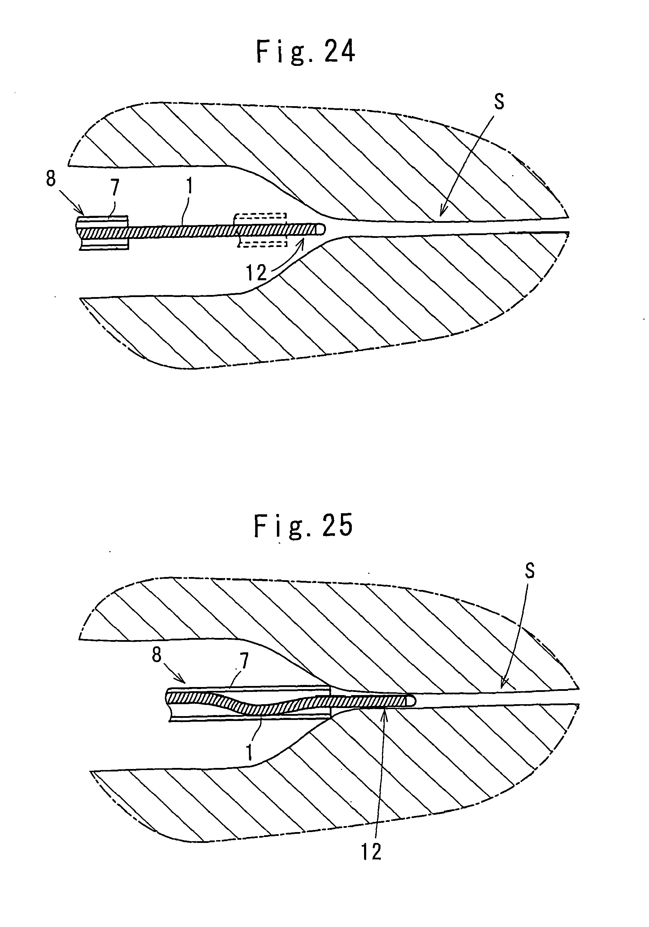

Figs. 24 and 25 show a sixth embodiment of the invention in which an assembly body 8 of the medical guide wire 1 and a microcatheter 7 is provided. The medical guide wire 1 is inserted into the microcatheter 7 upon using the assembly body 8 as shown in Fig. 24.

As reiterated hereinbefore, according to the invention, the medical guide wire 1 enables the operator to the deep insertion into the blood vessel with the use of the buoyance of the floatage chamber 5 and the increased pressure resistance, while at the same time, insuring the lightweight structure by utilizing the double-layered structure (synthetic resin layer 4) of different specific gravities.

This mitigates the mechanical properties (torque transmissibility, etc.,) needed for the medical guide wire 1, thus making it possible to render the medical guide wire 1 diametrically thin.

However, it may require a sufficient mechanical properties to pass the medical guide wire 1 through a highly occluded area S of the vascular system. In this instance, it can be mechanically insufficient for the thinned guide wire 1 to pass through the highly occluded area S.

In order to satisfy the mechanical properties for the medical guide wire 1, the microcatheter 7 is used. The microcatheter 7 is made of a flexible tube, an inner diameter of which is substantially small in scale.

After reaching the distal end portion 12 of the medical guide wire 1 at an entrance of the highly occluded area S as shown in Fig. 24, the microcatheter 7 is inserted near to the highly occluded area S as shown in Fig. 25.

Then, the medical guide wire is pushed with its proximal end portion (outside the patient's body) supportively held by the operator. At this time, by sharing the pushing force of the medical guide wire 1 with the microcatheter 7 as a reactionary force, it is possible to strengthen the pushability so as to supply the medical guide wire 1 with the forward propelling force.

In this situation, an outer diameter of the medical guide wire 1 is approx. 0.2032-0.254 mm (0.008-0.010 inches), and an inner diameter and outer diameter of the microcatheter 7 are in turn approx. 0.280-0.80 mm (0.0110-0.0315 inches) and 0.4-1.2 mm (00.157-00.47 inches).

With the assembly body 8 of the medical guide wire 1 and the microcatheter 7, although the medical guide wire 1 is diametrically thinned, it is possible to deeply insert the medical guide wire 1 into the blood vessel with the good pushability effectuated, thus coping with the demand of the low intrusiveness so as to lighten the burden of the patient when therapeutically treated.

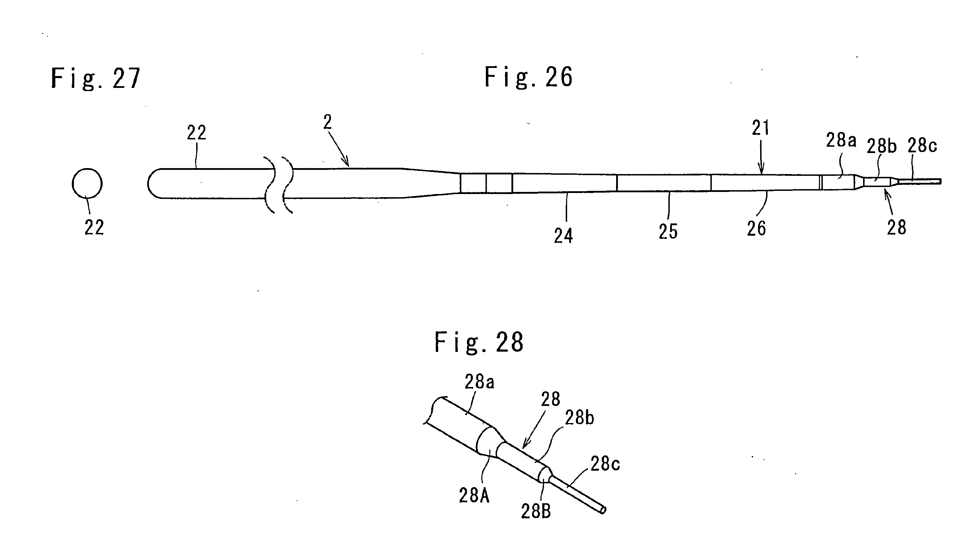

Figs. 26 through 28 show a modification form of the invention in which a multi-stepped tip portion 28 is provided at a topmost head of the distal end portion 21 in the elongate core 2.

The elongate core 2, a side elevational view of which is shown in Fig. 27, is structurally the same as described in the first embodiment of the invention (Fig. 5) except for the multi-stepped tip portion 28.

The multi-stepped tip portion 28 has outer diameters which decrease in three steps through stepped portions 28A, 28B as approaching the topmost head of the distal end portion 21, so as to form a first, second and third segment 28a, 28b, 28c each circular in cross section as shown in Figs. 26, 28.

When the multi-stepped tip portion 28 is manipulatively bent, the segments 28a, 28b, 28c represent their radius of curvature which progressively decreases in this order.

The multi-stepped tip portion 28 makes it possible to curvedly deform the distal end portion 21 within a narrow range, thus enabling the operator to staunchly follow the distal end portion 21 along the tortuous path of the vascular stricture area.

The multi-stepped tip portion 28 structurally differs from the multi-stepped flat portion 27 (Fig. 5) in that the multi-stepped flat portion 27 decreases its thickness dimension in three steps toward the topmost head of the distal end portion 21.

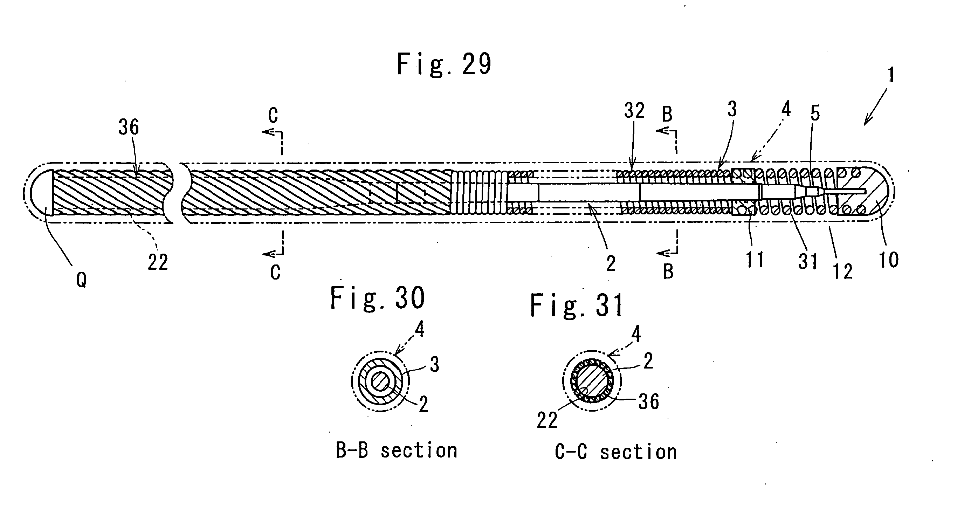

Figs. 29 through 31 show another modification form of the invention in which the radiotransparent helical portion 32 connects its proximal end to a wire-stranded helical spring 36. The wire-stranded helical spring 36, which is formed by a multitude of helically stranded wires, closely surrounds the proximal end portion 22 of the elongate core 2 as shown in Figs. 29, 31. The radiotransparent helical portion 32 surrounds the elongate core 2 in the manner as shown in Fig. 30.

The wire-stranded helical spring 36 fixedly secures its rear end to the rear end of the elongate core 2 by means of the soldering or welding procedure as designated at denotation Q in Fig. 29.

When comparing a general solid core to the combination of the wire-stranded helical spring 36 and the elongate core 2 in the condition that an outer diameter of the general solid core is identical to that of the wire-stranded helical spring 36, the combination makes its weight smaller than the weight of the solid elongate core because the wire-stranded helical spring 36 forms a concave groove between the neighboring coil lines of the wire-stranded helical spring 36.

Upon inserting the elongate core 2 into the blood vessel, the wire-stranded helical spring 36 permits the blood streams to flow along the concave groove, thus supplying a forward propelling force with the elongate core 2 so as to enable the operator to the deep insertion into the occluded area of the vascular system.

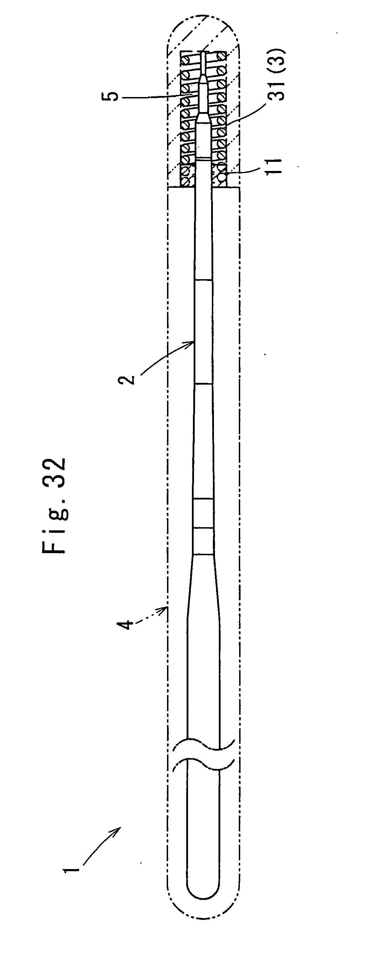

Fig. 32 shows still another modification form of the invention in which only the radiopaque helical portion 31 and the hermetical wall 11 remain to surround the elongate core 2. Other than an area which the radiopaque helical portion 31 and the hermetical wall 11 occupy in the elongate core 2, the synthetic resin layer 4 is coated on the outer surface of the elongate core 2.

In this modification form, it is possible to obtain the same advantages as mentioned in the first embodiment of the invention.

**Claims**

1.  A medical guide wire (1) in which is to be inserted into blood vessel, **characterised in that**
    a helical spring body (3), a distal end portion of which has a radiopaque helical portion (31) made by a radiopaque material, and a proximal end of which has a radiotransparent helical portion (32) made by a radiotransparent material;
    an elongate core member (2) placed within said helical spring body (3), and having a distal end portion (21) diametrically thinned and having a proximal end portion (22) diametrically thickened;
    a distal end of said helical spring body (3) and a distal end of said elongate core member (2) being air-tightly bonded;
    a synthetic resin layer (4) air-tightly coated on an outer surface of said helical spring body (3) in a circumferential direction;
    a hermetical wall (11) provided at a proximal end of said radiopaque helical portion (31) so as to air-tightly bond said helical spring body (3) and said elongate core member (2) together;
    a floatage chamber (5) provided within said radiopaque helical portion (31) by said hermetical wall (11), said synthetic resin layer (4) and a securement portion (10) in which said distal end of said helical spring body (3) and said distal end of said elongate core member (2) are air-tightly bonded;
    said synthetic resin layer (4) being lubricious in moistened condition more than in desiccated condition in which said synthetic resin layer (4) forms a cylindrical film (43) at a clearance (C) between neighboring coil lines (W) of said helical spring body (3) in a condition that an outer diameter of said cylindrical film (43) is smaller than that of said helical spring body (3) when expanding said helical spring body (3) in the moistened condition so that said clearance (C) between said neighboring coil lines (W) of said helical spring body (3) is 50-100 % of an outer diameter of said coil lines (W) of said helical spring body (3); and
    said helical spring body (3) having a central diameter portion (M) which is an average of an inner and outer diameter of said helical spring body (3);
    said cylindrical film (43) residing between an inner surface of said helical spring body (3) and said central diameter portion (M) of said helical spring body (3), so that an outer surface of said helical spring body (3) undulates to form a concave-convex portion (6) with said neighboring coil lines running as a convex portion (61) and said cylindrical film (43) deformed as a concave portion (62), so as to strengthen a forward propelling force due to a pressure resistance caused by blood streams colliding against said convex portion together with spiral currents caused by a coil-winding configuration of said helical spring body (3) when inserting said helical spring body (3) into a blood vessel.

2.  The medical guide wire (1) according to claim 1, which utilises an elastical restitution force developed by a pneumatic pressure increased within said floatage chamber (5) upon bending said radiopaque helical portion (31) of the helical spring body (3) into a bent position, and said pneumatic pressure decreased to an original quantity level within said floatage chamber (5) upon releasing said radiopaque helical portion (31) from said bent position, while at the same time, said forward propelling force is strengthened due to said pressure resistance caused by the blood streams colliding against said convex portion together with said spiral currents caused by said coil-winding configuration of said helical spring body (3) when inserting said helical spring body (3) into the blood vessel.

3.  The medical guide wire (1) according to claim 1 or 2, in which said synthetic resin layer (4) is made from a mixture of a hydrophilic polymer and a hydrophobic polymer, said synthetic resin layer (4) having a specific gravity decreasing from an inner side to an outer side of said synthetic resin layer (4) when moistened.

4.  The medical guide wire (1) according to claim 1, 2 or 3, in which said synthetic resin layer (4) has a first hydrophobic layer (41) on an outer surface of said helical spring body (3) as a solid layer and a second hydrophilic layer (42) as a fluid layer arranged on an outer surface of said first hydrophobic layer (41), a specific gravity of said second hydrophilic layer (42) being smaller than that of said second hydrophilic layer (42) when moistened.

5.  The medical guide wire (1) according to any of claims 1 to 4, in which said helical spring body (3) has said radiopaque helical portion (31) made of said radiopaque material, a spring-back quantity of which is smaller than that of a stainless steel wire.

6.  The medical guide wire according to any of claims 1 to 4, in which said helical spring body (3) has said radiopaque helical portion (31) made of said radiopaque material, a spring-back quantity of which is smaller than that of a stainless steel wire, said radiotransparent helical portion (32) being made of said stainless steel wire, and an outer diameter of said radiopaque helical portion (31) being smaller than that of said radiotransparent helical portion (3 2).

7.  The medical guide wire (1) according to any preceding claim, in which said helical spring body (3) is formed by connecting said radiopaque material to said radiotransparent material to produce a wire line element which is lengthwisely stretched to be diametrically thinned and helically wound to serve as a helical coil structure.

8. The medical guide wire (1) according to any preceding claim, in which said helical spring body (3) has a multitude of line wires, at least one of which contains said radiopaque material at a distal end portion of said helical spring body (3).

9. The medical guide wire (1) according to any preceding claim, in which said floatage chamber (5) encapsulates a foamy body (51) as a discrete foamy structure.

10. The medical guide wire (1) according to any one of claims 1 to 8, in which said floatage chamber (5) encapsulates globular foamy beads (53), a specific gravity of which ranges from 0.06 to 0.5.

11. An assembly body (8) of a microcatheter (7) and the medical guide wire (1) according to any preceding claim, in which an outer diameter of said medical guide wire (1) is approx. 0. 2032-0.254 mm (0.008-0.010 inches), and an inner diameter of said microcatheter (7) is approx. 0.280-0.80 mm (0.0110-0.0315 inches).

12. An assembly body of a guiding catheter, a balloon catheter and the medical guide wire (1) according to any one of claims 1 to 10, in which said medical guide wire (1) is inserted into said guiding catheter and said balloon catheter is guided by said guiding catheter, an outer diameter of said medical guide wire (1) is approx. 0.2032-0.254 mm (0.008-0.010 inches), an inner diameter of said balloon catheter is approx. 0.38-0.90 mm (0.015-0.032 inches) and an inner diameter of said guiding catheter is approx. 1.7-2.0 mm (0.067-0.079 inches).

Fig. 1

Fig. 2

Fig. 4

Fig. 3

A-A section

Fig. 5

EP 1 875 941 A1

Fig. 6

Fig. 7

Fig. 8

1

10

distal side

12

proximal side

Fig. 9

43 (4)

W

C

W

1

31 (3)

61

62

6

## Fig. 10

## Fig. 11

18

## Fig. 12

## Fig. 13

## Fig. 14

## Fig. 15

# Fig. 16

# Fig. 17

Fig. 18

Fig. 19

Fig. 21

Fig. 20

## Fig. 22

## Fig. 23

# Fig. 24

# Fig. 25

Fig. 27                    Fig. 26

22        2          21      28a  28b  28c

○

22                          24      25    26    28

Fig. 28

28a
                28    28b
28c

28A    28B

EP 1 875 941 A1

# Fig. 29

# Fig. 30

B-B section

# Fig. 31

C-C section

EP 1 875 941 A1

EP 1 875 941 A1

Fig. 32

European Patent
Office

**EUROPEAN SEARCH REPORT**

Application Number

EP 07 25 1897

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/041204 A1 (KATO TOMIHISA [JP]) 23 February 2006 (2006-02-23) | 1-7,9,10 | INV. A61M25/09 |
| Y | * paragraphs [0023], [0033], [0066], [0067], [0069] - [0072], [0080], [0081], [0105], [0110], [0113], [0126] * <br><br> * figures 1,10 * <br> ----- | 8,11,12 | |
| Y | DE 198 23 414 A1 (EPFLEX FEINWERKTECH GMBH [DE]) 17 June 1999 (1999-06-17) * figure 1 * * column 2, lines 23-26 * <br> ----- | 8 | |
| Y | US 5 676 659 A (MCGURK ERIN [US]) 14 October 1997 (1997-10-14) * claim 10 * <br> ----- | 11,12 | |
| Y | EP 1 023 913 A1 (KANEGAFUCHI CHEMICAL IND [JP]) 2 August 2000 (2000-08-02) * paragraph [0080] * <br> ----- | 12 | **TECHNICAL FIELDS SEARCHED (IPC)** |
| A | US 4 534 363 A (GOLD PHILIP [US]) 13 August 1985 (1985-08-13) * figure 3 * <br> ----- | 1-12 | A61M |
| A | US 2003/023190 A1 (COX BRIAN J [US]) 30 January 2003 (2003-01-30) * figures 1,2 * <br> ----- | 1-12 | |

The present search report has been drawn up for all claims

| Place of search | Date of completion of the search | Examiner |
|---|---|---|
| Munich | 14 September 2007 | Türkavci, Levent |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**                    EP 07 25 1897

This annex lists the patent family members relating to the patent documents cited in the above-mentioned European search report.
The members are as contained in the European Patent Office EDP file on
The European Patent Office is in no way liable for these particulars which are merely given for the purpose of information.

14-09-2007

| Patent document cited in search report | | Publication date | Patent family member(s) | | Publication date |
|---|---|---|---|---|---|
| US 2006041204 | A1 | 23-02-2006 | JP<br>JP | 3810413 B2<br>2005278795 A | 16-08-2006<br>13-10-2005 |
| DE 19823414 | A1 | 17-06-1999 | NONE | | |
| US 5676659 | A | 14-10-1997 | CA<br>DE<br>DE<br>EP<br>ES<br>JP<br>WO | 2176389 A1<br>69432359 D1<br>69432359 T2<br>0744977 A1<br>2194896 T3<br>9507399 T<br>9513110 A1 | 18-05-1995<br>30-04-2003<br>24-12-2003<br>04-12-1996<br>01-12-2003<br>29-07-1997<br>18-05-1995 |
| EP 1023913 | A1 | 02-08-2000 | AU<br>CA<br>CN<br>DE<br>DE<br>WO<br>US | 9458398 A<br>2307021 A1<br>1274294 A<br>69828429 D1<br>69828429 T2<br>9917831 A1<br>6706010 B1 | 27-04-1999<br>15-04-1999<br>22-11-2000<br>03-02-2005<br>08-12-2005<br>15-04-1999<br>16-03-2004 |
| US 4534363 | A | 13-08-1985 | NONE | | |
| US 2003023190 | A1 | 30-01-2003 | US | 2005004560 A1 | 06-01-2005 |

For more details about this annex : see Official Journal of the European Patent Office, No. 12/82

**REFERENCES CITED IN THE DESCRIPTION**

**Patent documents cited in the description**

- JP 2000135289 A
- JP 4009162 A
- JP 2588582 B